(19) Europäisches Patentamt
European Patent Office
Office européen des brevets

(11) EP 2 216 083 A2

(12) **EUROPEAN PATENT APPLICATION**

(43) Date of publication:
**11.08.2010 Bulletin 2010/32**

(21) Application number: **10153814.8**

(22) Date of filing: **28.06.2004**

(51) Int Cl.:
***B01D 15/08*** *(2006.01)* ***B01J 20/32*** *(2006.01)*
***C07K 1/22*** *(2006.01)* ***G01N 30/02*** *(2006.01)*
***C08F 8/32*** *(2006.01)*

(84) Designated Contracting States:
**AT BE BG CH CY CZ DE DK EE ES FI FR GB GR HU IE IT LI LU MC NL PL PT RO SE SI SK TR**

(30) Priority: **27.06.2003 GB 0315131**
**08.12.2003 GB 0328397**
**15.04.2004 GB 0408456**

(62) Document number(s) of the earlier application(s) in accordance with Art. 76 EPC:
**04743113.5 / 1 638 662**

(71) Applicant: **Invitrogen Dynal AS**
**0309 Oslo (NO)**

(72) Inventors:
- **Songe, Pal**
  **N-1176, Oslo (NO)**
- **Fonnum, Geir**
  **N-1472, Fjellhamar (NO)**
- **Kjus, Nini Hofslokken**
  **N-0679, Oslo (NO)**
- **Sandberg, Marcel**
  **N-0349, Oslo (NO)**
- **Nordstrand, Solveig**
  **N-0349, Oslo (NO)**
- **Aukrust, Inger Reidun**
  **N-0349, Oslo (NO)**

(74) Representative: **Harrison Goddard Foote**
**Belgrave Hall**
**Belgrave Street**
**Leeds**
**LS2 8DD (GB)**

Remarks:
•Claims filed after the date of receipt of the application (Rule 68(4) EPC).
•This application was filed on 17-02-2010 as a divisional application to the application mentioned under INID code 62.

(54) **Conjugates of magnetic polymer particles and carboxymethylated aspartic acid**

(57) A conjugate comprising a magnetic polymer particle bound to a carboxymethylated aspartate chelating ligand, optionally chelating a metal ion.

EP 2 216 083 A2

## Description

This invention relates to magnetic polymer particles carrying a chelating matrix loaded with a metal as well as to a process for the preparation of magnetic polymer particles carrying said chelating matrix. In particular, the invention relates to magnetic polymer particles carrying a carboxymethylated aspartate ,(Cm-Asp) chelating group and to the coupling of the Cm-Asp group with the magnetic polymer particle.

Magnetic polymer particles are of general utility in various medical and biochemical fields, for example as transport vehicles for the delivery of pharmaceutical products, for diagnostic purposes, for separation and for synthetic purposes. Such particles rely upon their magnetic properties in order to perform these functions: in diagnostic assay applications, for example, application of a magnetic field to a sample containing an analyte bound to magnetic polymer particles allows the isolation of the analyte without the use of centrifugation or filtration; and in therapeutic applications, for example, application of a magnetic field to the patient may serve to target drug-carrying magnetic polymer particles to a desired body site.

By magnetic is meant herein that the polymer particles contain superparamagnetic crystals. Thus the magnetic polymer particles are magnetically displaceable but are not permanently magnetizable. Many processes for preparing magnetic polymer particles are known, a large number of which involve preparing maghemite- or magnetite-containing polymer particles from pre-formed magnetic iron oxides, e.g. magnetite. Some of processes involved are described in US-A-4,654,267 (Ugelstad) the contents of which are incorporated herein by reference.

The use of immobilised metal ion affinity chromatography (IMAC) has been known for many years. The IMAC purification process is based upon the employment of a chelating matrix loaded with transition metal ions such as $Cu^{2+}$ or $Ni^{2+}$ which is capable of binding electron donating groups present on the surface of proteins, in particular the imidazole side chain of histidine. The electron donating group is believed to coordinate to vacant coordination sites around the metal ion. The interaction between the metal ion and the electron donating groups present on the protein surfaces can be altered by, for example, varying pH and hence purification can be achieved via reversible metal complex/protein interaction. Most commonly, if a protein is bound to a solid phase via the interaction between the metal ion and the imidazolyl side chain of histidine, the protein can be removed by addition of imidazole itself, i.e. by competitive elution.

Several different chelating ligands have been employed in IMAC to purify proteins. Nitrilo triacetate (NTA) (a tetradentate ligand) and the pentadentate ligand tris(carboxymethyl)ethylenediamine are examples of such ligands but these suffer from various disadvantages such as unspecific protein interaction, metal leakage etc.

US 6242581 proposes a solution to the metal leakage problem by the use of a carboxymethylated aspartate (Cm-Asp) group in IMAC where the bound transition metal ion has octahedral geometry. The ligand is said to be ideal for-isolating histidine tagged recombinant proteins. Other advantages of Cm-Asp are discussed in US 5962641, e.g. resistance to reducing agents.

In these Patents the Cm-Asp ligand is bound to an agarose solid phase which is preferably cross-linked although other polymer matrices such as polystyrene, nylon and SEPHAROSE are suggested. Whilst these matrices may be magnetic the magnetic particles do not remain in suspension and the solid phases are therefore of limited use in assays.

It has now been surprisingly found that the Cm-Asp chelating ligand can be coupled to a magnetic polymer particle giving rise to a moiety that possesses the ability to bind histidine-tags in recombinant proteins or His, Cys, Met, Gln, Asn, Lys or Tyr residues present in metalloprotein active sites in native proteins or peptides, the ability to bind phosphorylated proteins or peptides and also magnetism. This allows the skilled biochemist more flexibility in his assaying procedures.

The inventors have also devised ways to couple the Cm-Asp ligand to the magnetic polymer particles in high yield thereby producing an excellent IMAC agent.

Viewed from a first aspect, therefore, the present invention provides a conjugate comprising a magnetic polymer particle bound to a carboxymethylated aspartate chelating ligand.

Viewed from another aspect the invention provides a conjugate comprising magnetic polymer particle bound to a carboxymethylated aspartate ligand chelating a metal atom or ion.

Viewed from another aspect the invention relates to a process for the preparation of a conjugate as hereinbefore defined comprising reacting a magnetic polymer particle with a Cm-Asp chelating ligand.

The Cm-Asp ligand bound to the magnetic polymer particle (MPP) is depicted below both in its uncoordinated state and coordinated to a metal (the wavy line representing a bond or a linker between the Cm-Asp and particle) :

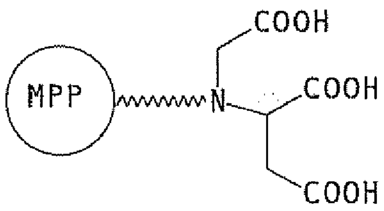

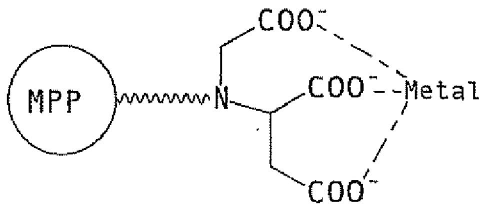

The magnetic polymer particles used in the process of the invention may be any magnetic polymer particle e.g. as described in US-A-4,654,267. The particles are preferably porous to allow the presence of the superparamagnetic crystals in the pores thereof. The surface of the magnetic particles is normally functionalised to allow coupling of the Cm-Asp ligand to the polymer particle, e.g. it may be functionalised to carry any known surface structure such as carboxyl groups, tosyl groups, amino groups, epoxy groups, maleamido groups, thiol groups etc. Hence, the surface may be amine functionalized before Cm-Asp coupling. Alternatively, an amine functionalised surface can itself be further functionalised to carry other functional groups, e.g. COOH groups.

The polymer particle is preferably made from combinations of vinylic polymers (e.g. styrene), acrylates and/or methacrylates. The polymeric material may optionally be crosslinked, for example by incorporation of cross-linking agents, for example as comonomers, e.g. divinylbenzene (DVB) or ethyleneglycol dimethacrylate. Appropriate quantities of the cross-linking agents (e.g. comonomers) required will be well known to the skilled man. Preferably the polymer is a cross-linked styrenic polymer (e.g. a styrenedivinylbenzene polymer, surface functionalized by the use of a nitro-group containing comonomer, e.g. nitro-styrene, and subsequent reduction) or a cross-linked (meth)acrylic polymer surface functionalized by the use of an epoxy-group containing comonomer (e.g. glycidylmethacrylate) and subsequent amination (e.g. by reaction with ethylene diamine).

The superparamagnetic crystals in the polymer particles used in the process of the invention may be of any material capable of being deposited in superparamagnetic crystalline form in the porous polymer particles. Magnetic iron oxides, e.g. magnetite or maghemite are preferred; however the crystals may be of mixed metal oxides or other magnetic material if desired. The total quantity of crystalline magnetic material present is generally more than 1%, preferably more than 3%, desirably more than or equal to 5% (by weight, e.g. up to 40% wt. The percentage is calculated on a Fe (or equivalent metal in the case of magnetic materials other than iron oxides) weight basis based upon the overall dry weight of the coated particles.

Polymer particles according to the various aspects of the present invention will generally have sizes (i.e. diameters) that are generally in the micrometer range, e.g. 0.3 to 100 μm, especially 0.5 to 50 μm, more especially 0.8 to 8 μm, e.g. 0.8 to 1.2 μm.

Typically the porous particles used will have a surface area of at least 15 $m^2/g$ (measured by the BET nitrogen absorption method), and more preferably at least 30 $m^2/g$, e.g. up to 700 $m^2/g$, when corrected to a mean particle diameter of 2.7 μm (i.e. multiply surface area by 2.7/MD, where MD is the mean diameter in micrometers). Similarly scaled, the particle pore volume is preferably at least 0.1 mL/g.

Typically, the polymer particles are spherical and substantially monodisperse before they are coated and especially preferably remain spherical and substantially monodisperse once they have been coated.

By substantially monodisperse it is meant that for a plurality of particles (e.g. at least 100, more preferably at least 1000) the particles have a coefficient of variation (CV) of less than 20%, for example less than 15%, preferably less than 12%, more preferably less than 11%, still more preferably less than 10% and most preferably no more than about 8%, e.g. 2 to 5%. CV is determined in percentage as

$$CV = \frac{100 \text{ x standard deviation}}{\text{mean}}$$

where mean is the mean particle diameter and standard deviation is the standard deviation in particle size. CV is preferably calculated on the main mode, i.e. by fitting a monomodal distribution curve to the detected particle size distribution. Thus some particles below or above mode size may be discounted in the calculation which may for example

be based on about 90% of total particle number (of detectable particles that is). Such a determination of CV is performable on a Coulter LS 130 particle size analyzer.

Functionalisation of the polymeric material may take place after polymerisation by, for example, nitration and subsequent reduction of the thus-formed nitro groups to pendant amine groups; or direct amination, for example by treatment with amino ethanol. As further alternatives, polymeric particles prepared by the well-known Ugelstad two-step swelling process and the improvements thereto disclosed in WO 00/61647 (Dyno) may be used. Porous polymer particles produced according to the processes described in this publication may have magnetic particles deposited in their pores by standard techniques.

As a further possibility, porous polymer particles may be prepared from nitro styrene and DVB, and magnetic material introduced as taught in US-A-4,654,267.

The superparamagnetic polymer beads sold by Dynal Biotech ASA under the trade names Dynabeads, especially Dynabeads MyOne are especially preferred. Dynabeads are particularly advantageous since they remain in suspension and do not exhibit magnetic particle sedimentation often associated with other magnetic beads. Dynabeads also show excellent magnetic mobility compared to other magnetic particles in which high levels of iron are present. Dynabeads exhibit beneficial kinetics allowing shorter reaction times and higher throughputs. Their unspecified binding is lower than other magnetic beads and their proper use results in a concentration of the desired material taking place resulting in easier and more efficient washing procedures. Finally Dynabeads, e.g. MyOne beads are easy to automate and are monodisperse.

The Cm-Asp ligand is bound to the magnetic polymer particle. By bound is meant that the ligand is covalently linked to the polymer particle, optionally using a linking group as discussed in detail below. The Cm-Asp ligand can be bound to the magnetic polymer particle by various procedures although it is preferred if there are at least three linking atoms between the polymer particle surface and the nitrogen atom of the Cm-Asp. Preferably there are at least 5 atoms separating the Cm-Asp ligand from the magnetic polymer particle surface, more preferably there are between 6 and 20 atoms separating the Cm-Asp ligand from the magnetic polymer particle surface.

The atoms forming the surface of the magnetic polymer particle are those on the surface just prior to coupling with the linker/Cm-Asp ligand. Thus, if the magnetic polymer particle is activated in some way during its manufacture (e.g. nitrated and reduced to form an amino functionalised surface) the magnetic polymer particle surface is formed by the surface nitrogen atoms. The nitrogen atom would form the first atom of the linker between the Cm-Asp ligand and the particle. In a magnetic particle functionalised to carry an electrophilic surface, e.g. a bromide surface, the first atom of the linker would be that which displaces the bromine.

Hence, in a preferred embodiment the invention provides a conjugate of formula (I)

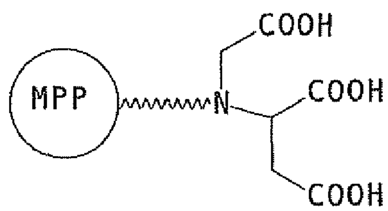

(where MPP is a magnetic polymer particle and the wavy line represents a linking group comprising at least three atoms, e.g. 3 to 20 atoms) or an analogue thereof in which a metal ion is chelated.

In US 6242581 aspartic acid is coupled to the solid phase prior to carboxymethylation to form the Cm-Asp ligand however it has not been possible to use this technique to provide a Cm-Asp group on a magnetic polymer particle. Rather, the inventors have devised alternative syntheses in which the Cm-Asp ligand is fully formed prior to coupling to the magnetic polymer particle.

In this regard, it has been found that when there are fewer than 3 atoms between the polymer surface and Cm-Asp ligand then coupling yields are low. In contrast to an agarose support carrying Cm-Asp (as describe in US-A-5962641), it is necessary in the present invention to ensure that coupling yields between the magnetic polymer particle and Cm-Asp are relatively high. The surface area of an agarose support is considerably greater than that of a polymer particle and hence the binding of Cm-Asp to the (agarose) support does not need to be achieved in high yield for a useful IMAC chelating agent to result. In the present case, yields need to be much higher to ensure that enough polymer particles carry the Cm-Asp ligand and hence to ensure that IMAC can be successfully carried out.

It is preferred if the at least 3 atom linker comprises an amino group (-NH-). Magnetic polymer beads are often made from styrene polymers which are nitrated to form $NO_2$, groups on the surface. After reduction of these groups, e.g. using ammonia, amino groups are formed.

The linker thus preferably comprises the residue of an electrophile, i.e. the group which remains after reaction

of the electrophile with a nucleophile. Hence, the linker may comprise an oxo group (C=O, the residue of an ester/carboxyl group), a $-CH(OH)CH_2-$ group (the residue of an epoxide), $-CH_2-$ (where the electrophile is, for example a $CH_2Hal$). The linker may also incorporate a number of atoms linking the actual electrophilic group to the nitrogen atom of the Cm-Asp ligand, e.g. an alkylene chain or ether chain, e.g. as in $-CH_2CH_2CH_2-$, or $-CH_2CH_2CH_2-O-$.

Hence the wavy line in formula (I) can represent - NH-Er-N- wherein Er represents a 2 to 20 atom linker which is an electrophile residue (Er), e.g. $-(CH_2)_n-$ where n is 2 to 20, which links the amino group of the particle surface with the nitrogen atom of the Cm-Asp ligand.

It is of course within the scope of the invention for the magnetic polymer particle to carry an electrophilic group with the Cm-Asp being functionalised to carry a nucleophilic group. Other ways of coupling the particle and ligand will be devised by the skilled chemist.

In an especially preferred embodiment, a particle coating is provided which carries a carbon-carbon double bond. This can be achieved by, for example, reaction of the particle with an allyl or vinyl compound, e.g. butenoic acid. Hydroxy functionalised particle surfaces can be reacted with allyl bromide to form double bonds on the particle surface. Also, carboxy functionalised particle surfaces can be reacted with allylamines to provide double bonds on the particle surface. The Cm-Asp may then be coupled directly to the double bond using appropriate chemistry or more preferably, the double bond may then be reduced e.g. in the presence of aqueous halide to provide a halide electrophile which can be reacted with the Cm-Asp ligand to ensure successful coupling.

Another preferred preparation process involves functionalising the surface of the magnetic polymer particle to carry carboxyl groups. The carboxylic acid groups can be activated by reaction with N-hydroxysuccinimide esters and reacted with a Cm-Asp ligand as discussed above.

Suitable linkers therefore include aminoalkylene, amidoalkylene, ether, ester, thioalkylene or thioester containing up to 20 atoms, e.g. NH-alkylene, NH-CO-alkylene, O-alkylene, OCO-alkylene, S-alkylene or SCO-alkylene. The nitrogen atom of the Cm-Asp ligand does not form part of the linker.

The Cm-Asp ligand may too be functionalised prior to coupling with the magnetic polymer particle. For example, it has proved advantageous to provide the Cm-Asp ligand with a linking group carrying a primary nucleophile to aid reaction with electrophilic groups on the particle surface. The nitrogen atoms of the Cm-Asp ligand is secondary and it has been found that this atom is too unreactive, perhaps due to steric hindrance, to react in high yield with electrophilic groups, e.g. halides, on the particle surface.

It is preferred therefore to couple the Cm-Asp to a linker group having at least two atoms and comprising a nucleophile such as an amine, hydroxyl or thiol group. Preferably the linker is an alkylamine, e.g. C5/6-alkylamine linker or an ether/polyether linkage e.g. comprising one or two oxygen atoms and 3 to 6 carbon atoms. Coupling of the linker to the Cm-Asp (via the nitrogen atom thereof) is achieved using known chemistry as described in the Examples. The Cm-Asp ligand itself can be manufactured using known chemistry. It is also possible to synthesise the entire linker CmAsp structure.

Thus, the linker CmAsp structure can be prepared starting from a suitably protected aspartic acid compound, e.g. where the carboxyl groups are ester protected. This compound can be reacted with a compound of formula Hal-$X_1$-CN (where X represents a $C_{1-9}$ alkylene group, and Hal a halide, e.g. Br) wherein the amino group of the aspartic acid derivative displaces the halide atom. The resulting secondary amino compound may then be reacted with a Hal-$CH_2COOPr$ type group (where Hal is halide, e.g. Br and Pr a protecting group) to introduce the final methylenecarboxy group to form the Cm-Asp structure. Selective reduction of the nitrile, e.g. using hydrogen and platinum (IV) oxide results in an ideal linker which can subsequently be deprotected as necessary.

Thus, viewed from a further aspect the invention provides a process for the preparation of a compound of formula

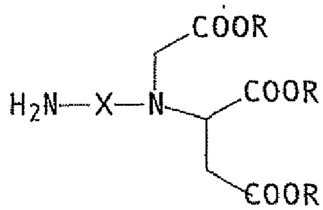

(wherein each R independently represents hydrogen or a protecting group and X represents an $C_{2-20}$ alkylene linker, especially a $C_{5/6}$-alkyle) ;

comprising reacting a compound of formula Hal-$X_1$-CN (wherein Hal is a halide and $X_1$, represents an $C_{1-19}$ alkylene linker) with a compound of formula

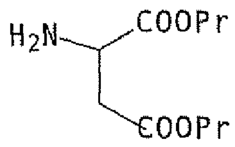

(wherein Pr represents a protecting group)
reacting the resulting product with a compound of formula Hal-$CH_2COOPr$ to form a compound

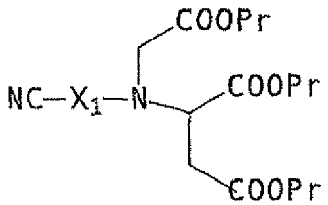

and reducing the nitrile to an amino group, preferably without removing the protecting groups. These can then be removed as necessary. The skilled chemist will realise that the X linker has one more carbon atom than the x linker which derives from the nitrile.
skilled chemist will be able to devise further for synthesising the Cm-Asp linker molecules of be invention.
wed from another aspect the invention provides a for the preparation of a conjugate comprising a polymer particle bound to a Cm-Asp ligand ng reacting a Cm-Asp ligand of formula (II)

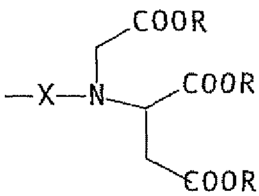

(II)

(wherein each R independently represents hydrogen or a protecting group and X represents a 2 to 20 atom linker, e.g. an $C_{2-10}$ alkylene linker, especially a $C_{5/6}$-alkylene linker) with a magnetic polymer particle, e.g, one functionalised to carry an electrophilic coating, e.g. an ester, epoxide, allyl, alkyl halide etc coating.

Compounds of formula (II) and analogues thereof in which a metal is chelated are themselves new and form a further aspect of the invention along with the Cm-Asp ligand itself, i.e. a compound of formula (III) and its analogue where a metal is chelated.

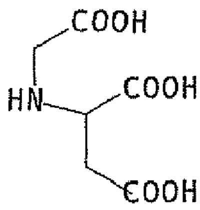

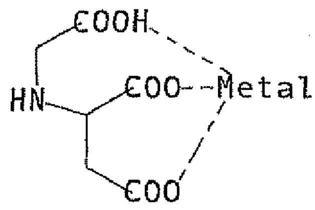

In some embodiments of the invention it may be necessary to protect the carboxyl groups of the Cm-Asp ligand

during syntheses. This can be easily effected using known protection strategies, e.g. using an ester protecting group which can be hydrolysed in acid or base as is known in the art.

The Cm-Asp ligand can coordinate any metal atom or ion. By metal is meant any metal from groups 1 to 13 of the periodic table, a lanthanide or actinide or a metal Si, Ge, Sn, Pb, As, Sb, Bi, Te, Po or At. The metal should preferably be an ion and preferably be a transition metal or a metal of group 13. Preferred metal ions are those in the 2+ or 3+ oxidation states. Where the metal ion is in the 2+ oxidation state, the entire particle-linker-ligand-metal ion assembly is uncharged which reduces the possibility of non-specific binding.

Preferred metals are Ni, Fe, Ga, Mn, Co, Cu and Zn of which Fe, Ga, Mn and Co are preferred, especially $Co^{2+}$: Coordination can be easily effected by exposing the Cm-Asp to, for example, a metal chloride.

The conjugates with associated metal can in general be used for attaching to and combining with peptides, proteins or other polymers (e.g. antibodies) and are hence of use in a wide variety of assays. They are of particular use, however, in the isolation of proteins/peptides tagged or native by immobilised metal ion affinity chromatography. In particular they are of use in the isolation of histidine-tagged recombinant proteins/peptides, His, Cys, Met, Gln, Asn, Lys and/or Tyr-containing native proteins and/or peptides, and phosphorylated proteins or peptides. Especially preferably, the conjugatges are of use in the isolation of histidine-tagged recombinant proteins/peptides. Hence viewed from another aspect the invention provides the use of a conjugate comprising a magnetic polymer particle bound to a Cm-Asp ligand, said ligand coordinating a metal atom or ion, in an assay. Suitable assays and ways to carry these out are known by the skilled biochemist.

For example, the capture of histidine-tagged on the Cm-Asp functionalised particles of the n has various applications. The rapid reaction and gentle handling of isolated proteins make hnology well suited for the "pull down" of large complexes. Thus Cm-Asp functionalised beads may in sample preparation for mass spectrometry It is believed that complexes isolated with sp functionalised beads may be more intact than s isolated with columns or other solid supports g other magnetic particles with uneven surfaces therefore ideal for use in mass spectrometry solation.

Cm-Asp technology may also act as a solid phase for use in assay procedures. The Cm-Asp beads are not prone to aggregation and are highly dispersed in solution and show a low degree of non-specific binding. These properties allow for high quality screening results and protocols that are easily automated on a wide range of automation platforms. The Cm-Asp beads may also be used in phage display perhaps as a solid phase or to purify expressed phage display selected proteins from a library.

In general therefore the capture of histidine tagged proteins and/or His, Cys, Met, Gln, Asn, Lys and/or Tyr residue containing native proteins or peptides may allow microscale protein purification, clean up of mutated protein libraries, denaturing elution of protein/peptide, mild elution of proteins/peptide, protein-protein interaction studies and screening technologies, e.g. for drug discovery, molecular display, aptamer screening, phage display, engineered enzyme screening and diagnostics.

The invention will now be described further by reference to the following non-limiting examples.

**Example 1**

Synthesis of the Cm-As ligand

The Cm-Asp triester below is prepared as follows:

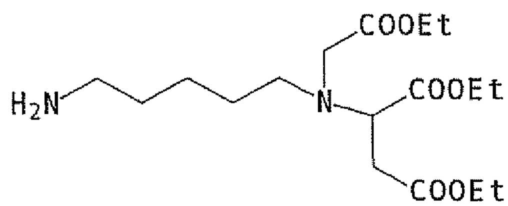

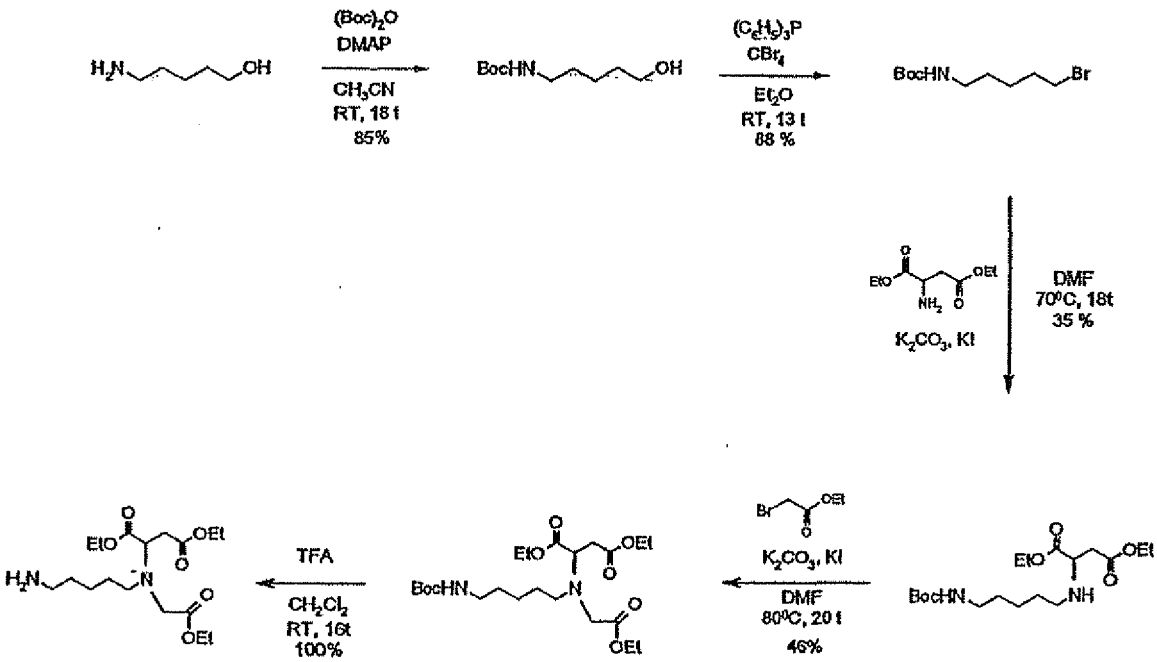


## Example 2

Alternative Synthesis of Cm-Asp triester

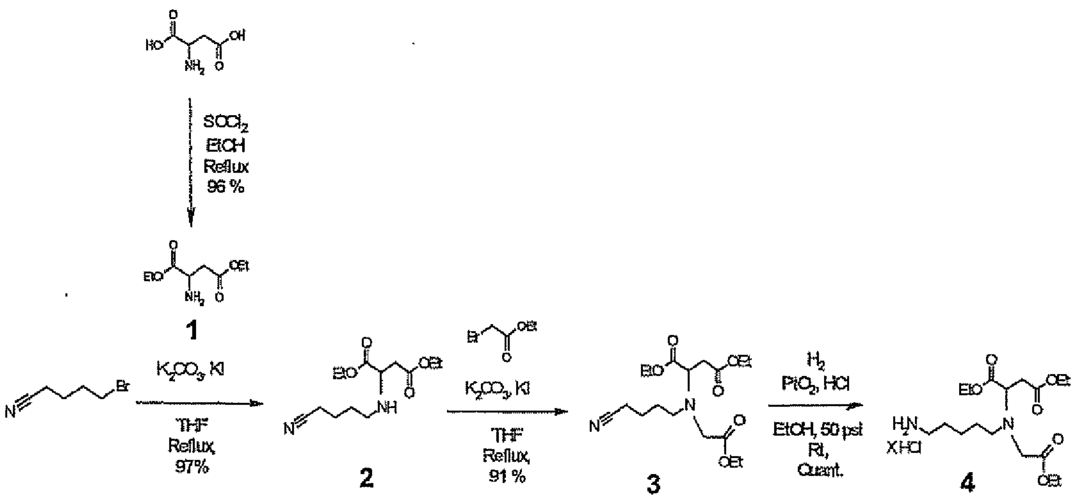


**Synthesis of 2-amino-succinic acid diethyl ester**

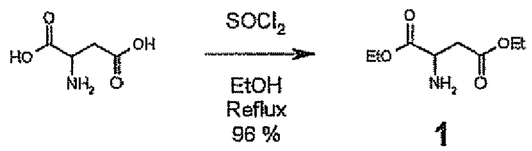


1

To a suspension of DL-aspartic acid (91.5 g, 0.69 mol) in abs. ethanol (800 ml) at 0°C thionylchloride (150 ml, 2.06 mol) was added dropwise. The cooling bath was removed and the mixture refluxed for 3 hours. After cooling to ambient temperature the solvent was evaporated *in vacuo* and to the residue added a saturated aqueous solution of $K_2CO_3$ to pH 8. The aqueous phase was extracted with ethyl acetate (x 3) and the combined organic phases washed with brine and dried ($MgSO_4$), prior to filtration and evaporation *in vacuo* to give 124.8 g (96 %) of compound 1 as an yellow oil. The crude product was used directly in the next step.

$^1H$ NMR (200 MHz, $CDCl_3$): 4.06 (m, 4H), 3.68 (m, 1H), 2.61 (m,2H), 1.73 (s, 2H), 1.06 (m, 6H).

**Synthesis of 2-(4-cyano-butylamino)-succinic acid diethyl ester**

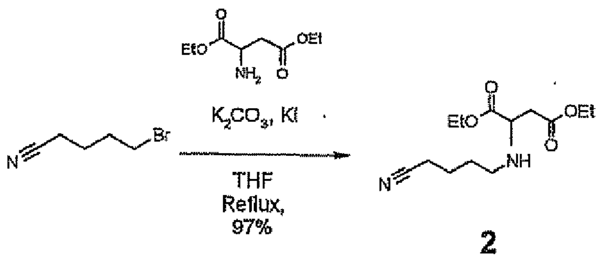


2

To a suspension of 1 (93.0 g, 0.49 mol), $K_2CO_3$ (34.0 g, 0.25 mol), and KI (12.3 g, 0.07 mol) in THF (600 ml) 5-bromovaleronitrile (28.4 ml, 0.25 mol) was added dropwise. The reaction mixture was heated to reflux and stirred for 5 days. After cooling to ambient temperature the mixture was filtered, and the filtrate evaporated *in vacuo.* Purification on silica gel, eluting with hexane/ethyl acetate (7:3) afforded 64.1 g (97 %) of compound 2 as an yellow oil.

$^1H$ NMR (200 MHz, $CDCl_3$) : 4.06 (m, 4H), 3.40 (t, 1H), 2.50 (m, 4H), 2.25 (t, 2H), 1.45 (m, 4H), 1.15 (m, 6H).

**Synthesis of 2-[(4-cyano-butyl)-ethoxycarbonylmethyl-amino]-succinic acid diethyl ester**

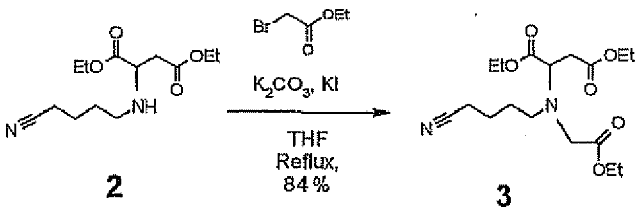


2

3

To a mixture of 2 (86.6 g, 0.32 mol), $K_2CO_3$ (44.3 g, 0.32 mol), and KI (16.0 g, 0.10 mol) in THF (650 ml) ethyl bromoacetate (42.5 ml, 0.38 mol) was added. The reaction mixture was heated to reflux and stirred for 5 days. After cooling to ambient temperature the mixture was filtered, and the filtrate evaporated in *vacuo.* Purification on silica gel, eluting with hexane/ethyl acetate (8:2) afforded 103.7 g (91 %) of compound 3.

$^1H$ NMR (200 MHz, $CDCl_3$): 4.18 (m, 6H), 3.91 (t, 1H), 3.42 (s, 2H), 2.77 (m, 4H), 2.40 (t, 2H), 1.65 (m, 4H), 1.25 (m, 9H).

**Synthesis of 2-[(5-amino-pentyl)-ethoxycarbonylmethyl-amino]-succinic acid diethyl ester**

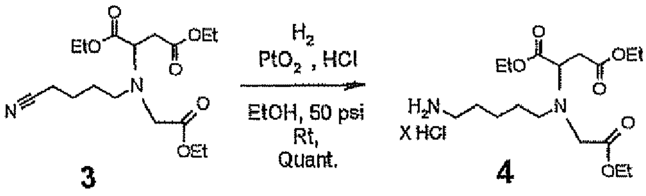


To a solution of **3** (15 g, 42 mmol) in 95% ethanol (60 ml) and concentrated HCl (10 ml) a suspension of $Pt0_2$ (600 mg, 2.6 mmol) in 95% ethanol (20 ml) was added. The reaction mixture was hydrogenated at 50 psi overnight. The mixture was filtrated and the filtrate evaporated in *vacuo* and pumped overnight to afford a quantitative yield of the title compound as the HCl-salt.

$^1H$ NMR (200 MHz, $D_2O$) : 4.82 (t, 1H), 4.20 (m, 6H), 3.53 (q, 4H), 3.34 (m, 2H), 3.18 (b d, 2H), 2.92 (b t, 2H), 1.65 (m, 4H), 1.10 (b m, 9H).

**Example 3:**

Bromination

17.3 g of a methanol suspension of the magnetic styrene particles having 0.5 mmol/g allyl groups were washed four times with 45 mL sodium acetate buffer (pH = 5.9). After adjusting the particle content to 9 wt%, 0.96 g of pyridinium tribromide dissolved in 10 mL DMF was added while stirring at 350 rpm. After five minutes at room temperature the particles were washed five times with 45 mL deionised water.

**Example 4:**

Functionalization with Cm-Asp chelator

18.0 g of a suspension of the particles prepared as in Example 3 were washed three times with 20 mL of 50mM sodium bicarbonate. The particle content was adjusted to 12 wt%. To the suspension 0.17 g of the Cm-Asp triester (prepared as described in Example 1) was added. 50mM sodium bicarbonate was added until a particle content of 10 wt% was achieved. The reaction mixture was shaken at 600 rpm at 40°C for 15 hours. The particles were then washed four times with 20 mL deionised water.

**Example 5:**

Hydrolysis

20.0 g of a suspension of particles prepared as in Example 4 were washed twice with 20 mL of 1M lithium hydroxide. After adjusting the particle content to 10 wt% the mixture was shaken at 250 rpm for four hours at room temperature. The particles were then washed with deionised water until pH 6-7.

**Example 6 :**

General Metal-loading conditions

250 mg of particles prepared as in Example 5 are washed twice with 5 ml reverse osmosis-water followed by 15 min sonication. 5 ml of 10 mM metal salt (MX) are added to the particles and incubated for 30 min. The tube is placed in a magnet, and the supernatant is removed. The particles are washed twice with 5 ml phosphate buffered saline (0,01% Tween 20, pH 7,4). The particles are then washed once in 20% ethanol. The particles are stored in 20% ethanol.

The following metal salts were employed MX = $CoCl_2$, $CuSO_4$, $FeCl_3$, $GaCl_2$, $GaCl_3$, $MnSO_4$, $MgCl_2$, $NiCl_2$, $CaSO_4$, $ZnCl_2$

**Example 7**

Functionalisation of carboxylic acid groups to N-hydroxysuccinimide ester

50 g of a suspension of 5.0 g of the particles of MyOne Carboxylic acid beads (Dynal Biotech ASA) were acidified by washing with 0.1 M acetic acid (3 x 50 mL). The acidified particles (which have a carboxylic acid content of 0.5 mmole/g DS) were then washed with acetone (4 x 50 mL) and concentrated on a magnet. Extra acetone was added until a total of 35.6 g suspension is achieved. N-hydroxysuccinimide (2.90 g, 25 mmole) and diisopropylcarbodiimide (3.16 g, 25 mmole) were then added. The reaction mixture was stirred at room temperature for 5 hours. The particles were then washed with acetone (5 x 50 mL).

**Example 8:**

Functionalization with Cm-Asp chelator

44 g of an acetone suspension of the beads of Example 7, were washed three times with 50 mL isopropanol. After adjusting the particle content to 12 wt%, 5.6 g of triethylamine was added. 0.10 g of the Cm-Asp triester (prepared as described in Example 1) dissolved in isopropanol, was then added. This results in a particle content of 10 wt%. The reaction mixture was then shaken at 250 rpm at room temperature for 20 hours. The particles were washed three times with 50 mL of isopropanol.

**Example 9:**

Functionalization with Cm-Asp chelator and ethanolamine

To 10 g of an isopropanol suspension of the particles prepared as in Example 8, 0.32 g of ethanolamine was added. The reaction mixture was then shaken at 250 rpm at room temperature for 18 hours. The particles were then washed three times with 10 mL of isopropanol.

**Example 10**

Functionalization with Cm-Asp chelator

1,2 gram of dry Dynabeads 270 Epoxy were mixed with 8,8 gram of 50 mM sodium bicarbonate. To the suspension 0,17 grams of the Cm-Asp triester (prepared as described in Example 1) were added, and the reaction mixture was shaken at 600 rpm at 60°C for 16 hours. The particles are worked up by washing four times with 20 ml deionised water.

**Example 11**

Purification of Histidine-tagged recombinant proteins

1. 2 mg of a suspension of particles with $Co^{2+}$ prepared as in Example 6 were washed with 700 μl 50 mM Na-phosphate, pH 8.0, 300 mM NaCl, 0.01% Tween®-20.
2. The supernatant was removed and the particles were resuspended in 100 μl of the same buffer as in step 1.
3. A suspension of lysed E. coli cells with expressed recombinant histidine-tagged protein was added to the particle suspension. The total volume was adjusted to 700 μl with the same buffer as in step 1. This suspension was incubated for 10 minutes.
4. The supernatant was removed and the particles with the bound histidine-tagged protein were washed four times with 700 μl of the same buffer as in step 1.
5. The histidine tagged protein was eluted in 100 μl 150 mM Imidazole, 50 mM Na-phosphate, pH 8.0, 300 mM NaCl, 0,01% Tween®-20.
6. The purified protein was analysed by SDS-Tris-HCl polyacrylamide gel and bromphenol blue staining.

**Example 12**

Purification of phosphorylated peptides

1. 2 mg of a suspension of particles with $Fe^{3+}$ prepared as in Example 6 were washed twice with 500 ml 5% acetic acid.
2. The supernatant was removed and 100 μl 10% acetic acid was added. 100 μl of 30 μg trypsinated b-casein was added. This was incubated for 30 min.
3. The supernatant was removed and the particles with the bound phosphorylated peptides were washed twice with 250 μl 1% acetic acid.
4. The supernatant was removed and the particles with the bound phosphorylated peptides were washed twice with 250 μl 0,1% acetic acid, 10% acetonitrile.
5. The supernatant was removed and the particles with the bound phosphorylated peptides were washed with 250 μl $H_2O$.
6. The phosphorylated peptides were eluted in 50 μl 0,1 M ammonium bicarbonate.
7. The purified phosphorylated peptides were analysed by HPLC.

**Example 13**

Purification of metal binding proteins

1. 2 mg of a suspension of particles with $Mn^{2+}$ prepared as in Example 6 were washed twice with 250 μl Acetate buffer pH 4.0, 250 mM NaCl.
2. The supernatant was removed and 250 ml of the same buffer as in step 1 was added. 30 μg (3 μl) of an Mn binding protein was added. This was incubated for 10 min.
3. The supernatant was removed and the particles with the bound protein were washed twice with 250 μl of the same buffer as in step 1.
4. The Mn-binding protein was eluted in 50 μl 0,1 M ammonium bicarbonate.
5. The eluted protein was analysed by SDS-Tris-HCl polyacrylamide gel and silver staining.

Further embodiments are described in the following paragraphs:

1. A conjugate comprising a magnetic polymer particle bound to a carboxymethylated aspartate chelating ligand.

2. A conjugate comprising a magnetic polymer particle bound to a carboxymethylated aspartate ligand chelating a metal atom or ion.

3. A conjugate as described in paragraph 2 wherein said metal is a transition metal or a metal of group 13.

4. A conjugate as described in paragraph 3 wherein said metal is Ni, Fe, Ga, Mn, Co, Cu and Zn.

5. A conjugate as described in paragraph 4 wherein said metal is Fe, Ga, Mn and Co.

6. A conjugate as described in paragraph 2 to 5 wherein said metal is in the 2+ or 3+ oxidation state.

7. A conjugate as described in paragraph 6 wherein said metal is $Co^{2+}$, $Fe^{3+}$, $Ga^{3+}$ and $Cu^{2+}$.

8. A conjugate as described in paragraph 7 wherein said metal is $Co^{2+}$.

9. A conjugate as described in any one of paragraphs 1 to 8 wherein there are at least three atoms between the nitrogen atom of the carboxymethylated aspartate ligand and the particle surface.

10. A conjugate as described in paragraph 9 being of formula

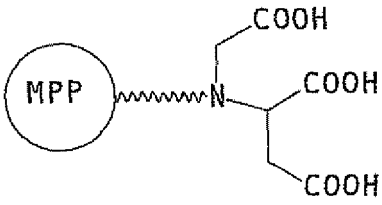 or 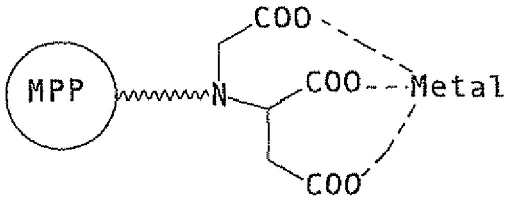

(MPP=magnetic polymer particle)
wherein the wavy line represents a 3 to 20 atom linker selected from NH-alkylene, NH-CO-alkylene, O-alkylene, OCO-alkylene, S-alkylene or SCO-alkylene.

11. A conjugate as described in paragaph 10 wherein the wavy line represents NH-$C_5H_{12}$- or NH-$C_6H_{13}$-.

12. A conjugate as described in any one of paragraphs 1 to 11 wherein said polymer comprises a cross-linked styrene divinyl benzene polymer.

13. A conjugate as described in any one of paragraphs 1 to 12 wherein the magnetic polymer particle has a diameter of 0.5 to 8 $\mu$m.

14. A conjugate as described in paragraph 12 wherein said magnetic polymer particle has a diameter of 0.8 to 1.2 $\mu$m.

15. A conjugate as described in any one of paragraphs 1 to 14 being uncharged.

16. A conjugate as described in any one of paragraphs 2 to 15 additionally chelated to a histidine tagged recombinant protein/peptide, His, Cys, Met, Gln, Asn, Lys and/or Tyr residue containing native protein/peptide or phosphorylated protein/peptide.

17. A conjugate as described in any one of paragraphs 2 to 16 additionally chelated to a histidine tagged recombinant protein/peptide.

18. A conjugate as described in paragraph 16 characterised in that where said conjugate binds a phosphorylated protein/peptide, said metal is Fe or Ga.

19. A process for the preparation of a conjugate comprising a magnetic polymer particle bound to a Cm-Asp ligand comprising reacting a Cm-Asp ligand of formula (II)

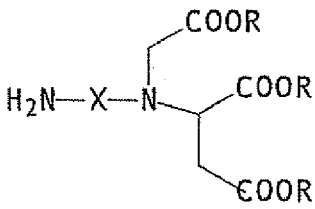 (II)

(wherein each R independently represents hydrogen or a protecting group and X represents a 2 to 20 atom group) with a magnetic polymer particle, and optionally coordinating the resulting conjugate to a metal atom or ion.

20. A compound of formula (II)

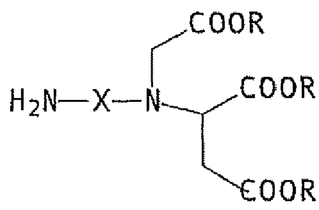

(wherein each R independently represents hydrogen or a protecting group and X represents a 2 to 20 atom group) or an analogue therefore in which the R groups are absent and a metal chelated.

21. A compound as described in paragraph 20 wherein X is a C5 or C6-alkylene group.

22. A compound of formula (III) or an analogue thereof in which a metal is chelated

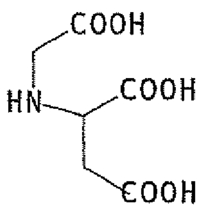

(III)

23. A process for the preparation of a compound of formula

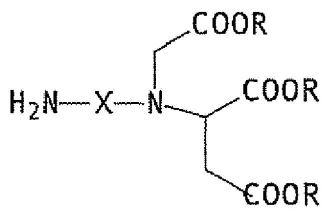

(wherein each R independently represents hydrogen or a protecting group and X represents an $C_{2-20}$ alkylene linker); comprising reacting a compound of formula Hal-$X_1$-CN (wherein Hal is a halide and $X_1$ represents an $C_{1-19}$ alkylene linker) with a compound of formula

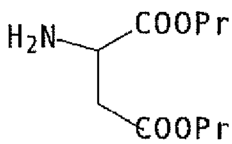

(wherein Pr is a protecting group)
reacting the resulting product with a compound of formula Hal-$CH_2$COOPr to form a compound

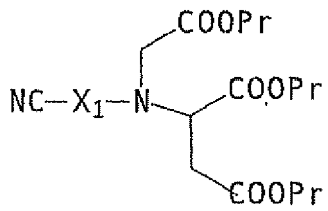

reducing the nitrile to an amino group; and optionally deprotecting the carboxyl groups.

25. Use of a conjugate as described in any one of paragraphs 2 to 18 in an assay.

26. Use of a conjugate as described in any one of paragraphs 2 to 18 in the purification of histidine tagged recombinant proteins/peptides, His, Cys, Met, Gln, Asn, Lys and/or Tyr residue containing native proteins/peptides or phosphorylated proteins/peptides.

## Claims

1. A compound of formula (II)

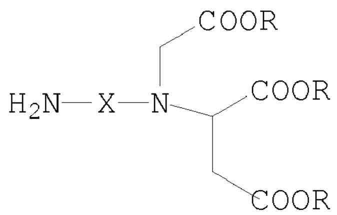

wherein each R independently represents hydrogen or a protecting group and X represents a 2 to 20 atom group or an analogue therefore in which the R groups are absent and a metal chelated,
provided that the compound is not ethylenediamine-N-carboxymethyl-N-monosuccinic acid.

2. A compound of claim 1 wherein X is $-(CH_2)_n-$, wherein n is from 2 to 20.

3. A compound of claim 1 wherein X is a C5 or C6-alkylene group.

4. A compound of claim 1 which is

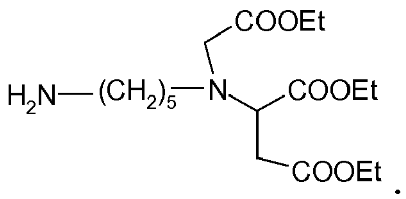

5. A compound of formula (III) or an analogue thereof in which a metal is chelated

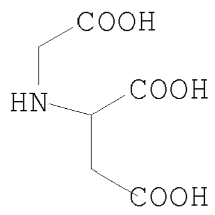

(III)

6. A process for the preparation of a compound of formula

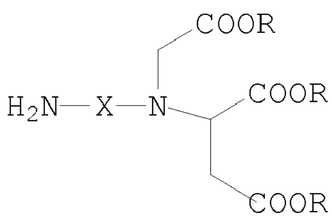

wherein each R independently represents hydrogen or a protecting group and X represents an $C_{2-20}$ alkylene linker; comprising reacting a compound of formula Hal-$X_1$-CN, wherein Hal is a halide and $X_1$, represents an $C_{1-19}$ alkylene linker, with a compound of formula

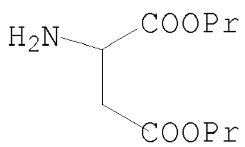

wherein Pr is a protecting group;
reacting the resulting product with a compound of formula Hal-$CH_2$COOPr to form a compound

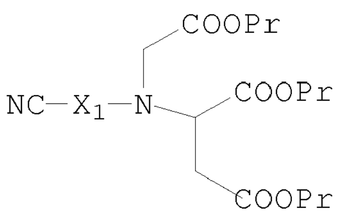

;

reducing the nitrile to an amino group; and optionally deprotecting the carboxyl groups.

7. The process of claim 6, wherein X is as defined in claim 2 or claim 3 or wherein the end product compound is as defined in claim 4.

8. The process of claim 6 or claim 7, which further comprises reacting the compound of formula

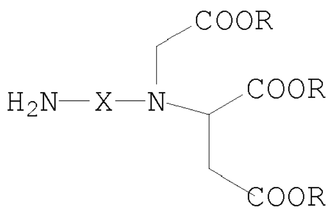

with a magnetic polymer particle, and optionally coordinating the resulting conjugate to a metal atom or ion.

**9.** The use of a compound of any of claims 1 to 4, when disregarding the disclaimer of claim 1, to couple a magnetic polymer particle carrying an electrophilic coating by reacting the compound with the particles.

**10.** The use of claim 9 wherein the R groups present during the reaction are protecting groups.

**11.** The use of claim 9 or claim 10 which further comprises coordinating the carboxy groups to a metal species selected from metal atoms and metal ions.

**12.** The use of claim 11 wherein the metal species is a metal ion in the 2+ or 3+ oxidation state.

**13.** The use of claim 11 or claim 12 wherein the metal is Ni, Fe, Ga, Mn, Co, Cu and Zn of which Fe, Ga, Mn or Co, in particular $Co^{2+}$.

**14.** A conjugate comprising a magnetic polymer particle bound to a compound of any of claims 1 to 4 wherein each R represents a protecting group, the particle and the compound being bound through a covalent linkage resulting from reaction of the amino group of the compound of any of claims 1 to 4 and an electrophilic group, optionally wherein the linkage comprises an oxo group (C=O, the residue of an ester/carboxyl group), a $-CH(OH)CH_2-$ group (the residue of an epoxide), $-CH_2-$(where the electrophile is, for example a $CH_2Hal$).

**15.** The use of a conjugate of claim 14 to make a product in which the -COOR groups are modified by removal of R and the conjugation of the groups to a metal species as defined in any of claims 11 to 13.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4654267 A, Ugelstad
- US 6242581 B
- US 5962641 A
- WO 0061647 A